Europäisches Patentamt

European Patent Office

Office européen des brevets

(19)

(11) Publication number: **0 511 559 A1**

# EUROPEAN PATENT APPLICATION

(12)

(21) Application number: **92106603.1**

(22) Date of filing: **16.04.92**

(51) Int. Cl.⁵: **C12Q 1/68**, C12P 19/34

(30) Priority: **30.04.91 US 694226**

(43) Date of publication of application:
**04.11.92 Bulletin 92/45**

(84) Designated Contracting States:
**AT BE CH DE DK ES FR GB IT LI NL SE**

(71) Applicant: **F.HOFFMANN-LA ROCHE & CO. AKTIENGESELLSCHAFT**
**Postfach 3255**
**CH-4002 Basel(CH)**

(72) Inventor: **Kawasaki, Ernest S.**
**2601 San Mateo Street**
**Richmond, California 94804(US)**
Inventor: **Levenson, Corey H.**
**311 Mandalay Road**
**Oakland, California 94618(US)**
Inventor: **Will, Stephen G.**
**2247 Ivy Drive**
**Oakland, California 94606(US)**
Inventor: **Zhang, Yong**
**1241 18th Avenue**
**San Francisco, California 94122(US)**

(74) Representative: **Notegen, Eric-André et al**
**Grenzacherstrasse 124 Postfach 3255**
**CH-4002 Basel(CH)**

(54) **Oligonucleotide probe reagent.**

(57) The invention relates an oligonucleotide probe reagent consisting of a nylon membrane with a high density of anionic carboxyl groups to which membrane at least one oligonucleotide probe containing a 5' amine is covalently bound via an amide bond and to a method for attachment of the oligonucleotide probe to the nylon membrane. The single, 5' end attachment of the oligonucleotides to the membrane surface leaves the probe free to interact with complementary sequences, thus increasing the hybridization efficiency relative to probes attached by methods in which heat or ultraviolet light is used for immobilization. The simplicity and reproducibility of this method and the sensitivity attained when using the reagents produced by the method are ideal for application of the method and reagents to the diagnosis of infectious and genetic diseases, the analysis of mutations in neoplasias, performance of HLA typing, and other areas.

EP 0 511 559 A1

The present invention relates to an oligonucleotide probe reagent, a method for constructing the oligonucleotide probe reagent and kits based thereon.

Generally the invention relates to the field of nucleic acid based diagnostics and particularly to methods for site-specific attachment of oligonucleotide probes to nylon membranes. The resulting membrane bound probes and probe arrays are useful in the diagnosis of infectious, genetic, and neoplastic diseases.

The polymerase chain reaction (PCR) technology (see U.S. Patent Nos. 4,683,195; 4,683,202; and 4,965,188) has been extensively applied in many areas of the biological sciences. PCR has had a dramatic impact on the ability of investigators to analyze very small quantities of DNA or RNA, even single molecules as described by Li et al., 1988, Nature 335:414-417. Because of this sensitivity, analysis of large numbers of samples for research and diagnostic purposes relating to infectious diseases, genetic disorders, cancer, etc., is now much easier.

When the sample size is large, a convenient format for analysis is the "dot blot," where the PCR products are bound to a filter membrane and hybridized with gene or allele specific probes (see e.g. EP Publication No. 237,362). When only a small number of probes is required, this method is quite satisfactory. However, if the number of probes is large, this method becomes cumbersome, because each allele or gene requires a separate probing for the analysis of the PCR products.

An alternative method called the "reverse dot blot" alleviates some of these problems. In this method, the probe is bound to the filter, and the PCR product is free in solution and hybridizes to the bound probe, hence the name, "reverse dot blot." Examples where this reverse format is useful are analysis of the cystic fibrosis (CF) locus and the RAS oncogenes. CF is one of the most common genetic diseases in Caucasian populations and more than 60 mutations have been found at this locus (see Roberts, 1990, Science 250:1076-1077). Transforming mutations of the RAS oncogenes are found quite frequently in cancers, and more than 60 probes are needed to detect the majority of the mutated variants (see Bos, 1989, Cancer Res. 49(17):46824689). Analyses of the CF or RAS mutants by conventional means is a difficult, complex, and daunting task. However, with the reverse dot blot format, a researcher can analyze all the mutations on one filter, with considerable savings in time and effort (see Saiki et al., 1989, Proc. Natl. Acad. Sci. 86:6230-6234; WO 89/11548).

The filters containing the immobilized array of probes must be prepared for this purpose, and there is a need in the art for a better attached oligonucleotide probe reagent and hence for more efficient and better methods for attaching oligonucleotides to activated membranes and for methods suitable for both the research and the clinical diagnostics laboratory.

The present invention meets that need in that the invention provides an oligonucleotide probe reagent consisting of a nylon membrane with a high density of anionic carboxyl groups to which membrane at least one oligonucleotide probe containing a 5' amine is covalently bound via an amide bond.

The oligonucleotide probe reagent is further characterized in that it contains a spacer. The nylon membrane is preferably a Biodyne C™ membrane or an equivalent one. In addition different probes can be attached to said membrane at a discrete location. It is preferred that a set of different probes is attached to the membrane so that each probe is present at a location different from the location of every other member of that set. Thereby at least one probe of that set can be a mixture of different probes. In preferred embodiments, said set of probes is composed of probes that can hybridize to DNA that contains a CF or a RAS mutation or comprises probes for analysis of cytochrome P450 systems and chromosomal translations.

A method for constructing the membrane-bound oligonucleotide probe reagent according to the present invention comprises (a) activating a nylon membrane with a high density of anionic carboxyl groups by treatment with a reagent that converts carboxyl groups to O-acylureas; and (b) reacting an oligonucleotide probe containing 5' amine with an O-acylurea formed in step (a) to attach the probe to the membrane via an amide bond form between said 5'-amine and said O-acylurea. A preferred reagent for converting carboxyl groups to O-acylureas in the method is 1-ethyl-3-(dimethylaminopropyl)carbodiimidehydrochloride (EDC). A preferred nylon membrane for use in the method is a Biodyne C™ (Pall Corp. Glen Cove, NY) membrane or an equivalent membrane thereto. In a preferred embodiment of the method, the membrane is treated, after step (b), to render inactive any unreacted O-acylureas formed in step (a) to a nucleophile. The treatment is preferably performed with hydroxylamine and/or NaOH, especially with 0.1N NaOH.

In especially preferred embodiments, a set of different probes is attached to the membrane so that each probe is present at a location on the membrane different from the location of every other probe of said set. Using the method, one preferably can attach a mixture of different probes to the membrane at a particular location, and such a mixture can serve as one probe of a set of different probes immobilized on a membrane in a manner such that each probe of the set is present at a location on the membrane different from the location of every other probe of the set.

The present invention provides a method for immobilizing probe(s) on a solid support and immobilized probe reagent(s) for diagnostic, forensic, and other applications. The method is especially preferred for detection of cystic fibrosis (CF) and RAS mutations. In these embodiments, the set of probes is composed of probes that can hybridize to DNA that contains a CF mutation or the set of probes is composed of probes that can hybridize to DNA that contains a RAS mutation. In similar fashion, analysis of the cytochrome P450 system and of diseases associated with chromosomal translocations, such as ALL, AML, and CML, can be facilitated with the method and reagents of the present invention.

In another aspect, the invention relates to kits comprising the probe reagents prepared and especially to kits comprising reagents useful in performing the afore mentioned method for making and/or using the probe reagents. Kits can comprise amplification reagents, i.e., if the preferred amplification method is PCR, then such reagents can be primers, nucleoside triphosphates, and/or polymerase. Kits for making the reagents can comprise nylon membranes, EDC, oligonucleotides, and/or quenching reagents.

Hereafter the present invention is explained by figures and examples.

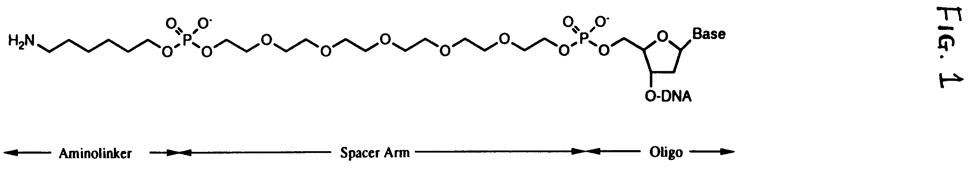

Figure 1 shows schematically the structure of the oligonucleotide amino-linker and spacer arm.

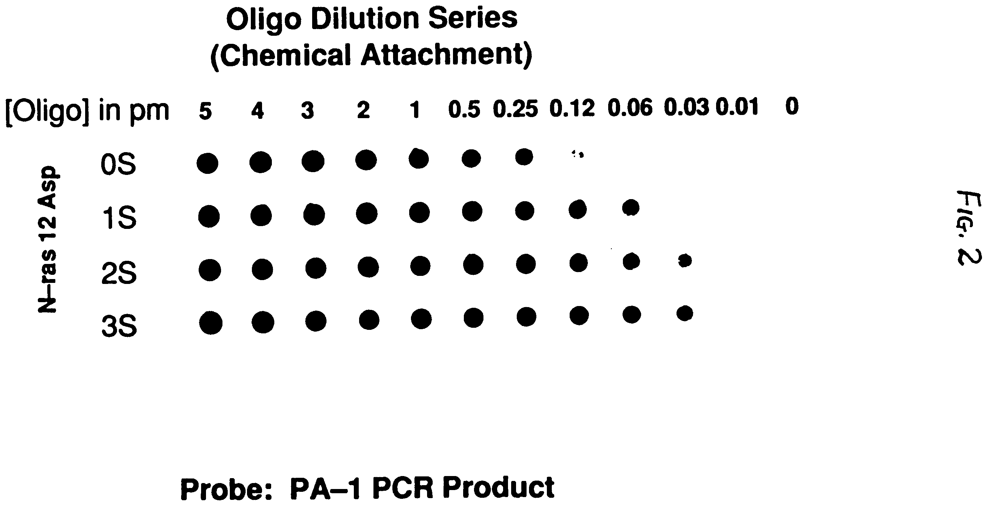

Figure 2 shows the results of a hybridization test of oligonucleotide probes with varying concentrations and spacer lengths. Probes (N-RAS 12 Asp (SEQ ID NO: 1) 5'-GAGCAGATGGTGTTGG) were diluted as described in Example 2, subsequently immobilized on the membranes, and hybridized to PCR amplified N-RAS 12 sequences from PA-1 DNA. "S" is spacer.

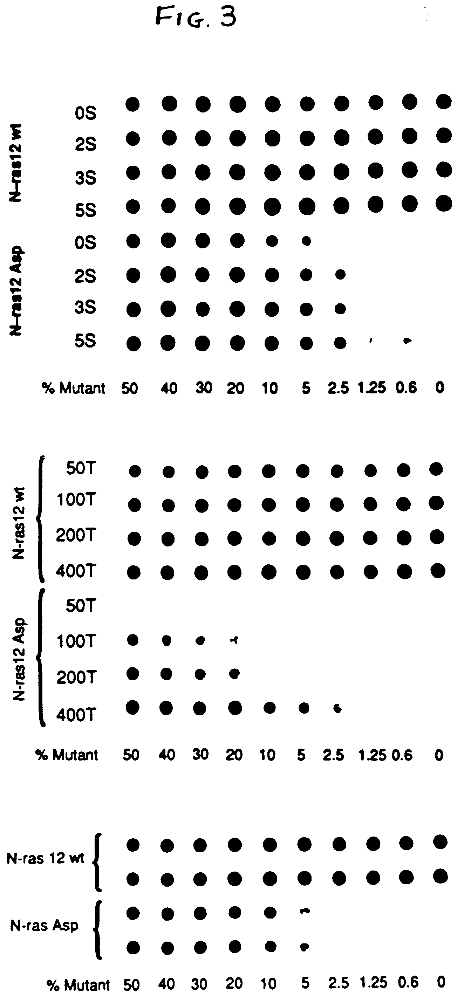

Figure 3 shows the sensitivity of the reverse dot blot format. Two pmoles of the oligonucleotides that represent the N-RAS 12 wild-type (wild-type, wt (SEQ ID NO:2) 5'-GAGCAGGTGGTGTTGG) and N-RAS 12 Asp mutant probes were immobilized on the membranes and hybridized to PCR amplified PA-1 DNA (heterozygous for N-RAS 12 Asp) that were serially diluted in normal PBL DNA such that the mutant sequence was represented from 50% to 0% (see Example 7): (a) aminomodified probes on a Biodyne C™ filter; (b) enzymatically tailed probes on a BiodyneB™ filter; and (c) chemically synthesized 100T tailed probes on a Biodyne B™ filter.

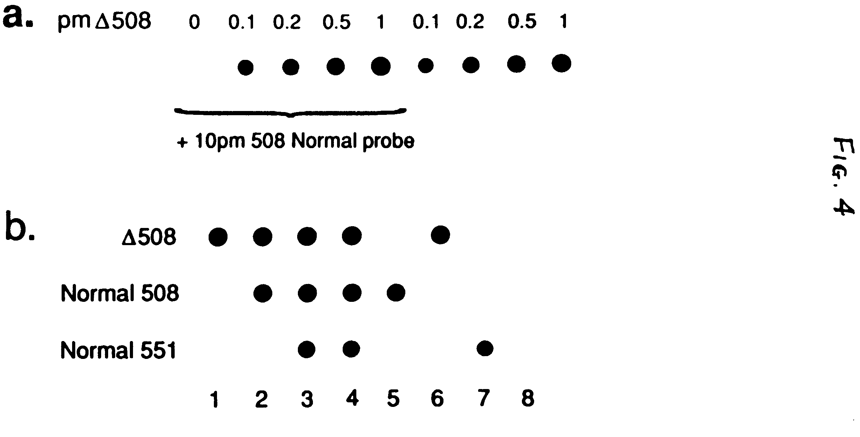

Figure 4 shows the results of pooling of oligonucleotide probes: (a) a probe that recognizes Δ 508 mutant of CF (Δ 508 (SEQ ID NO: 3) 5'-AATATCATTGGTGTTTCCTAT) was mixed with 10 pmoles of the wild-type CF probe (wt (SEQ ID NO: 4)5'-TATCATCTTTGGTGTTTCCTA) and hybridized with PCR product of DNA from a CF patient carrying 508 on both alleles; and (b) three identical Biodyne C™ filters with probes at position 1: Δ 508, 2: Δ 508 + wt 508, 3: Δ 508 + wt 508 + wt 551, 4: Δ 508 + wt 508 + wt 551 + mutant 551, 5: wt 508, 6: Δ 508, 7: wt 551, and 8: mutant 551. The probes were immobilized on Biodyne C™ membranes as described in Example 4. The probes were hybridized with PCR products of Δ 508 (row 1), wt 508 (row 2), and wt 551 (row 3), respectively.

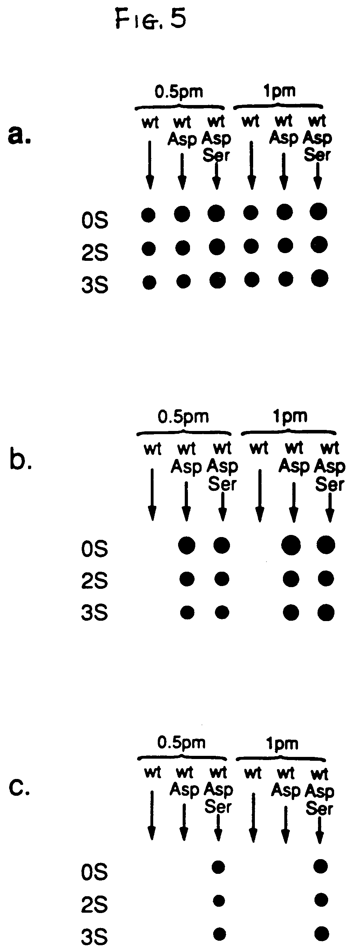

Figure 5 shows the detection of RAS point mutations in a mixture of oligonucleotides. Equal amounts of three RAS probes ((SEQ ID NO: 2),

## 5'-GAGCAGGTGGTGTTGG; (SEQ ID NO: 1), Asp: 5'-GAGCAGATGGTGTTGG; Ser (SEQ ID NO: 5), 5'-GAGCAAGTGGTGTTGG)

with a varying number of spaces were immobilized on Biodyne C™ membranes. The same filter was subsequently hybridized with HRP-labelled complementary strand oligonucleotides:

    a) wt (SEQ ID NO: 6)
5'-GTGCGCTTTTCCCAACACCACCTGCTCCAACCACCACCAG;
    b) Asp (SEQ ID NO: 7)
5'-CGCTTTTCCCAACACCATCTGCTCCAACCA; and
    c) Ser (SEQ ID NO: 8)
5'-CGCTTTTCCCAACACCACTTGCTCCAACCA

that recognize the respective mutant sequences as indicated.

The invention provides a method for immobilizing oligonucleotide probes on nylon membranes through a covalent binding reaction. The reaction is a chemically controlled coupling rather than the random attachment characteristic of photochemical crosslinking (UV). The immobilization is site-specific, and the covalent bonds formed between the probes and the surface molecules of the membrane are very strong. The membrane probes provided by the method are an important aspect of the invention and not only can withstand multiple stringent hybridizations and washings but also can be reused after being stripped by boiling in a microwave oven.

The attachment method provides a membrane bound probe reagent that provides excellent sensitivity in the reverse dot blot format; less than 0.1 pmole oligonucleotide probe per spot is sufficient to give a strong hybridization signal. At the same time, the binding capacity per dot is high, with more than 50 pmoles required to saturate an area equivalent to the size of one well in any common 96 well dot blot apparatus. This high sensitivity in combination with high capacity of attachment allows one to bind several different probes on one dot without sacrificing detection capabilities, which is very useful in the clinical diagnostic setting where large scale screening is often necessary for detecting and analyzing infectious and genetic diseases or cancer. These reagents have great potential in the study and analysis of HLA Class II genes. The HLA Class II loci are highly polymorphic, and classification of the polymorphisms is important in cases of organ transplantation, paternity, forensics, and in other areas. In marked contrast, normal serological or dot blot typing methods are much more difficult in these settings, because of the scarcity of reagents and the presence of many alleles in the population.

In the reverse dot blot format, all the bound probes on a filter should have approximately the same melting temperature ($T_m$). The invention provides reagents and methods for making membrane bound probe arrays in which the probes have $T_m$'s roughly within 2 centigrade degrees of each other. In cases where discrimination of single-base changes are required, TMAC1 (see Wood et al., 1985, Proc. Natl. Acad. Sci. USA 82:1585-1588) can be used in the washing buffer to minimize the differences among the probes caused by variations in the base composition of the oligonucleotides (see Example 6; RAS point mutation detection).

The construction of a membrane bound probe array reagent of the invention begins by activation of the nylon membrane. For solid phase type hybridizations, nylon membranes are a suitable substrate, because they are easy to handle, durable, and convenient for both hybridization and detection procedures. The membrane should have a high density of anionic carboxyl groups to be reactive with amino-modified oligonucleotides. Of the negatively charged membranes tested, only IAM (Millipore, MA) and Biodyne C™ membranes were able to bind oligonucleotides functionalized by introducing nucleophile groups, such as amines or thiol groups which are reactive with derivatized surfaces, at their 5' end during synthesis.

Oligonucleotides bind to IAM predominantly through hydroxyl groups when one uses the manufacturer's recommended conditions, whereas Biodyne C™ membrane binding is specifically through the primary amines of the modified end. Hydroxyl and sulfhydryl modified oligonucleotides have shown only a very low level of non-specific binding. Biodyne C™ is a preferred membrane because of the ability of its carboxyl groups to react with the amino-linkers of the oligonucleotides through EDC (see U.S. Patent No. 4,693,985). In addition, the weakly anionic property of Biodyne C™ membranes may contribute to the reduction of non-specific binding due to electrostatic interactions between the nucleic acids and the membranes. The chemical reactions for activating the Biodyne C™ membrane are simple and straightforward and do not require the use of organic solvents.

The nylon membrane is activated by treatment with EDC, which activates carboxyls to form an O-acylurea. These intermediates, in turn, can be attacked by amines to form amide bonds. The coupling efficiency depends upon the competition between hydrolysis and aminolysis of the O-acylurea. In principle, at least in liquid phase, both the amino-linker and the exocyclic amines of the bases can react with the activated carboxyl groups. However, the 5' primary amine is a stronger nucleophile than the aromatic amines on the bases. For purposes of the present invention, both reaction efficiency (speed) and selectively (specific amino-linker attachment) are desirable.

The coupling efficiency was tested as a function of (1) EDC concentration with the amount of oligonucleotides constant; and (2) oligonucleotide concentration with EDC concentration constant at 2 M. A covalent binding efficiency of 80-90% was achieved after 2 hours of immobilization. The percentage of end attachment of the amino-linker was calculated by the amount of binding of amino-modified oligonucleotides minus the amount of the binding of unmodified oligonucleotides (nonspecific binding) divided by the total amount of covalent binding. Various immobilization times yielded about 90% specific end attachment, higher than reported in a liquid phase reaction (see Ghosh et al., 1987, Nuc. Acids Res. 15:5353-5372). The high percentage of end attachment implies that the immobilization capacity, which is determined solely by the density of functional groups on the membrane surface, has not been exceeded. With both a high capacity and efficient attachment, it is much easier to immobilize a defined amount of oligonucleotide probe.

Non-specific binding through electrostatic, hydrophobic, or chemical interactions can reduce the sensitivity of an assay system dramatically. The activated carboxyls are reactive to any nucleophile present in the solutions. After oligonucleotide immobilization, it is important to block any remaining preactivated sites prior to subsequent use, because nucleic acid probes can attach to the sites at a low but significant level, even without an amino-linker.

Different quenching regents have been tested, including proteins (casein), bifunctional molecules containing a nucleophilic substituent (ethanolamine, hydroxylamine), amino acids (glycine), and 0.1 N NaOH, which can hydrolyze all the remaining active esters without affecting the covalent bond between the oligonucleotide probes and the membranes. Membranes were preactivated with EDC and then quenched with different reagents for varying times. Amino-modified oligonucleotides were applied to the quenched membranes to determine if they could still bind covalently. No binding of the probes was observed in membranes quenched by any of the reagents used. Hydroxylamine and 0.1 N NaOH gave the maximum efficiency of quenching in the shortest time. NaOH is preferred for effectiveness and simplicity.

The single, 5' end attachment of the oligonucleotides to the membrane surface leaves the probe free to interact with complementary sequences, thus increasing the hybridization efficiency relative to probes attached by methods in which heat or ultraviolet light is used for immobilization.

The hybridization efficiency of the membrane bound probes was studied using different amounts of membrane-immobilized oligonucleotides containing varying linker lengths. As Figure 2 demonstrates, a strong signal can be obtained by immobilizing as little as 0.06 pmoles of oligonucleotide with one or two spacers, while oligonucleotides without spacers are 4-fold less efficient in hybridization. The access of the target nucleic acid to the membrane-bound probes depends on the presence and the length of a linker. In a test in which a dilution series of a RAS mutant sequence was hybridized against complementary oligonucleotides with differing numbers of spacers (see Figure 3a and Example 7), the membrane bound probes with spacers resulted in a 2-4 fold increase in sensitivity as compared to methods using membrane bound probes with no spacers. Spacer length may be more important as target product size increases, i.e., longer spacers are preferred for larger products.

The present covalent binding method was compared with a poly T tail procedure (see Saiki et al., 1989, Proc. Natl. Acad. Sci. USA 86:6230-6234). T tailing of oligonucleotides was done by both enzymatic reaction and chemical synthesis, and immobilization was done by UV crosslinking on Biodyne B™ membranes. As Figures 3b and 3c show, with enzymatic tailing, addition of 400 T's is necessary to approach the sensitivity comparable with the covalently immobilized probes without a spacer. When oligonucleotides containing 100 chemically synthesized T's were tested, only 10 to 20% of the mutant sequence could be detected, an amount similar to the usual dot blot protocol using a $^{32}$P-labelled probe. With less target DNA than this, the signal approaches the background.

The sensitivity of diagnostic methods employing the present reagent was also compared to the conventional dot blot format, in which the PCR products are immobilized to the membranes and hybridized to a $^{32}$P- or biotin-labelled probe. With a radioactive probe, 10 to 20% of the DNA has to be mutant to be detected. With a nonradioactive probe labelled with the ECL light emitting system, the sensitivity approximates the reverse dot blot method, but the background is considerably higher. In addition, it is much easier to distinguish single base changes by the reverse dot blot method versus the conventional format. In the present format, both probe and PCR product are free to interact as if in solution without steric constraints. With probes covalently bound at their 5' ends, all probes hybridize with equal efficiency to the PCR products. In contrast, in the usual dot blot format, the PCR product is immobilized randomly, so that all target sequences may not be available for hybridization. In addition, the amount of PCR product applied to the filter in the conventional format is almost always variable, because the PCR reactions cannot be accurately controlled to synthesize the same amounts of target sequence. Thus, variable quantities of bound product can give variable hybridization signals, while the reverse format is much more consistent, and each spot contains a precise amount of probe.

The single, site-specific end attachment of the oligonucleotides provided by the present invention has made possible the immobilization of several different probes on one dot. In theory, all the probes should have equal probability of being bound and should all be freely available for hybridization. If probes are bound by UV or heat, then the probes will be attached in random fashion to the filter and sometimes to each other, making it more difficult to determine consistent or optimal hybridization conditions. Pooling of oligonucleotides can be very useful in the diagnostic test setting where a large number of probes is required to detect many mutations that are present in low frequency. Analyses of the CF and RAS loci are examples of this category of diagnostic tests; there are certain mutations present in high frequency in these systems, but most occur on the order of 1% or less. Probes for the low frequency mutation can be pooled in one dot if only a yes/no type answer is required. In addition, the size and the complexity of the test filters can be reduced by pooling.

In one test of the pooled probe reagent of the present invention, varying amounts of mutant specific probes were mixed with 10 to 20 pmoles of related sequences derived from separated exons or differing by only a single-base. The probe mixtures were bound to filters and hybridized with complementary PCR products or oligonucleotides. As shown in Figure 4, there was no problem in identifying the proper sequence even when mixed with a large excess of related or unrelated oligonucleotides. As little as 0.1 pmole of specific probe was sufficient to give a strong signal even in the presence of 10 pmoles of other probes, showing that up to a 100 fold excess of a different sequence can be used for a pooled dot.

In cases of many CF mutants, the yes/no type signals are perhaps easier to obtain, because the probe sequences can be very different from one another. For a more stringent hybridization test, three RAS oligonucleotide probes were immobilized on one dot (Figure 5). These probes differ by only one base and represent the possible sequence changes in codon 12 of N-RAS. As Figure 5 shows, with this more stringent test, a mixture of three probes with only a single base mismatch among them, the present reagent provides the ability to differentiate the single base changes in RAS point mutations.

The simplicity and reproducibility of this method and the sensitivity attained when using the reagents produced by the method are ideal for application of the method and reagents to the diagnosis of infectious and genetic diseases, the analysis of mutations in neoplasias, performance of HLA typing, and other areas.

Thus, the present invention provides useful methods and reagents for nucleic acid-based diagnostics. The invention is further exemplified below.

Example 1

The example relates to allele-specific oligonucleotide synthesis and 5'-modifications. Oligonucleotide syntheses were performed on a Milligen/Biosearch Model 8750 DNA synthesizer using reagents and protocols obtained from the manufacturer. The terminal primary amino groups ("aminolinkers") were introduced during the final coupling step on the DNA synthesizer using N-trifluoroacetyl-6-aminohexyl-2cyanoethyl N', N'-diisopropylphosphoramidite (see Beaucage et al., 1981, Tetra. Lett. 22:1859-1862, and Sinha et al., 1984, Nuc. Acids Res. 12:4539-4557), which was purchased from Milligen/Biosearch (Novato, CA). A hexaethylene glycol-based cyanoethyl diisopropyl phosphoramidite reagent (see U.S. Patent No. 4,914,210) was used to introduce the spacer groups during the penultimate coupling step on the machine (immediately prior to addition of the 5'-amino group). The combined length of the "spacer" and "aminolinker" would be approximately twenty-eight angstroms if the chain were fully extended (Figure 1).

The crude amino-labelled oligonucleotides were converted to their lithium salts by precipitation from 4 M lithium chloride using five volumes of cold ethanol:acetone 1:1. The precipitates were pelleted by brief centrifugation (15 minutes at 4000 G). The supernatants were decanted and discarded and the pelleted nucleic acid reconstituted in water and stored at -20°C. Oligonucleotides can be purified by PAGE and quantitated following elution from the gel by determination of the OD at 260 nm (see Borer, 1975, "Handbook of Biochemistry and Molecular Biology" 3rd edition, Nucleic Acids 1, Fasman, ed., CRC Press, p. 589). Neither the spacer nor the aminolinker contributes significantly to the absorbance at this wavelength. Oligonucleotides conjugated to either biotin or horseradish-peroxidase (HRP) were prepared as previously described (see Levenson et al., 1990, "PCR Protocols" Innis et al., eds., Academic Press, pp. 99-113).

Example 2

The example relates to oligonucleotide immobilization studies. The 5' amino-modified oligonucleotides were 3' end labelled with $^{35}$S-ddATP (du Pont NEN, specific activity, 1240 Ci/mmol, 12.5 $\mu$Ci/$\mu$l). A 50 $\mu$l reaction containing 50 pmoles of oligonucleotides, 10 $\mu$l of $^{35}$S-ddATP, 2 $\mu$l of TdT (terminal deoxyribonucleotidyl transferase, 12 U/$\mu$l, Pharmacia), and 5 $\mu$l of 10 x TdT buffer (1 M potassium cacodylate, 250 mM Tris-HCl, 10 mM $CoCl_2$, and 2 mM dithiothreitol, pH 7.6) was incubated at 37°C for 60 minutes. For binding studies, the labeled oligonucleotides were mixed with unlabeled oligonucleotides in a 1:50 ratio. The Biodyne C™ membranes (Pall Biosupport, NJ) were cut into a 96-well plate size and acidified by a rinse with 0.1 N HCl, then preactivated with EDC. Varying amounts of oligonucleotides were applied to preactivated membranes in sodium bicarbonate buffer (pH 8.4) for different lengths of time. The amount of immobilized oligonucleotide was calculated by the cpm as measured by Ambis-scan (Ambis Corp., San Diego).

Example 3

The example relates to DNA isolation and PCR amplification. Mutant N-RAS 12 genomic DNA was isolated by a salting-out method (see Miller et al., 1988, Nuc. Acids. Res. 16:1215) from PA-1 cell line (see Tainsky et al., 1984, Science 225:643-645) which harbors an N-RAS exon I, codon 12, G to A transition resulting in an amino acid substitution of glycine by aspartic acid.

DNA (0.5 $\mu$g) was amplified for N-RAS exon I region in a 100 $\mu$l reaction volume containing 50 mM KC1, 10 mM Tris-HCl, 1.5 mM $MgCl_2$, 0.2 mM each of dATP, dCTP, dTTP, and dGTP (Pharmacia), 30 pmol of each biotinylated amplification primer (see Farr et al., 1988, Proc. Natl. Acad Sci. USA 85:1629-1633) and 2.5 units of Thermus aquaticus (Taq) DNA polymerase from Perkin Elmer Cetus Instruments (PECI). The reaction was performed in a thermal cycler (PECI, Norwalk, CT) using the cycle: denaturing at 95°C for 30 seconds, annealing at 55°C for 30 seconds, and extending at 72°C for 30 seconds. After 35 cycles, the samples were incubated for an additional 10 minutes at 72°C.

For detection of cystic fibrosis mutations, the biotinylated PCR product was amplified from a patient who had DNA with CF mutations in codons 508 and 557 and a patient who was homozygous for the Δ 508 mutation.

Example 4

The example relates to the filter preparation and subsequent immobilization of the oligonucleotides. Biodyne C™ membranes were briefly rinsed with 0.1 N HCl, then treated for 15 minutes with freshly prepared 20% EDC (w/v) in deionized water, and rinsed with deionized water. The preactivated membrane was immediately placed in a 96-well Biodot apparatus (Bio-Rad, CA). Amino-modified oligonucleotides were applied to it in 0.5 M sodium bicarbonate buffer, pH 8.4, for 15 minutes. The dots were rinsed with TBS (Tris-Buffered Saline)/0.1% Tween-20. Any remaining active groups were quenched with 0.1 N NaOH for 10 minutes. Finally, filters were rinsed with deionized water and air dried for storage or immediately used for hybridization (prehybridization is not necessary).

Biodyne B™ membranes (Pall Biosupport, NJ) were used to immobilize poly Ttailed oligonucleotides. The methods of poly T tailing (chemically synthesized or enzymatically tailed) and immobilization are essentially the same as described by Saiki et al., supra.

Example 5

The example relates to the quenching of the preactivated membranes. Quenching reagents tested were (1) 10-20% ethanolamine in sodium bicarbonate buffer, pH 9.5, (2) 4 M hydroxylamine in sodium bicarbonate buffer, pH 9.5, (3) 3 M glycine in sodium bicarbonate buffer, pH 9.5, (4) 0.5-1% casein (Sigma) in TBS buffer, and (5) 0.1 N NaOH. Quenching reagents were tested separately for their ability to block active groups and were applied to the preactivated membranes for varying lengths of times prior to the oligonucleotide immobilization.

Example 6

The example relates to the hybridization and non-radioactive detection of the amplified DNA. Filters with immobilized oligonucleotides were placed in a plastic tray or sealed in plastic bags and hybridized either with HRP-labeled complementary probes or with denatured (0.25 N NaOH) biotinylated PCR products in 5 x SSPE/0.5% SDS (1 x SSPE = 180 mM NaCl, 10 mM $NaH_2PO_4$, 1 mM EDTA, pH 7.2) for 30 minutes at 45°C (RAS and CF). The filters were washed in 3 M TMAC1 (tetramethylammonium chloride; see Ghosh et al., 1987, Nuc. Acids Res. 15:5353-5372) at 50°C for 15 minutes (RAS) or in 2 x SSPE/0.1% SDS at 45°C (CF).

Filters with hybridized biotinylated products were incubated with 1-2 ml streptavidin-HRP (5 mg/ml, commercially available in the AmpliType® DQαDNA Typing Kit, available from Cetus, CA) in 2 x SSPE/0.1% SDS for 15 minutes, then washed in the same buffer for 10 minutes. Equal volumes of ECL Gene Detection reagents A + B (Amersham, IL) were applied to the filters for 1 minute, and the filters were removed, exposed to Hyperfilm-ECL (Amersham, IL) or Kodak XRP film for a few seconds or minutes.

Example 7

The example relates to the effect of a spacer arm. The teratocarcinoma cell line PA-1 is heterozygous for an N-RAS 12 mutation changing glycine to aspartic acid. Two pmoles of the oligonucleotides (asp = positive control for PA-1 mutation; gly = wild-type) were used. PA-1 DNA was serially diluted with normal peripheral blood leucocyte (PBL) DNA such that the mutant sequence represented from 50% to 0% of the DNA. The diluted samples were PCR amplified and hybridized against the various probes. As shown in Figure 3a, as little as 2.5% of mutant DNA (equal to 5% mutant cells) can be detected when the probes have spacers. This is 2-4 fold more sensitive than the probes without the spacer.

# SEQUENCE LISTING

- INFORMATION FOR SEQ ID NO:1:

    (i) SEQUENCE CHARACTERISTICS:
      (A) LENGTH: 16 base pairs
      (B) TYPE: nucleic acid
      (C) STRANDEDNESS: single
      (D) TOPOLOGY: linear

    (ii) MOLECULE TYPE: DNA (genomic)

    (xi) SEQUENCE DESCRIPTION: SEQ ID NO:1:

GAGCAGATGG TGTTGG             16

- INFORMATION FOR SEQ ID NO:2:

    (i) SEQUENCE CHARACTERISTICS:
      (A) LENGTH: 16 base pairs
      (B) TYPE: nucleic acid
      (C) STRANDEDNESS: single
      (D) TOPOLOGY: linear

    (ii) MOLECULE TYPE: DNA (genomic)

    (xi) SEQUENCE DESCRIPTION: SEQ ID NO:2:

GAGCAGGTGG TGTTGG             16

- INFORMATION FOR SEQ ID NO:3:

    (i) SEQUENCE CHARACTERISTICS:
      (A) LENGTH: 21 base pairs
      (B) TYPE: nucleic acid
      (C) STRANDEDNESS: single
      (D) TOPOLOGY: linear

    (ii) MOLECULE TYPE: DNA (genomic)

    (xi) SEQUENCE DESCRIPTION: SEQ ID NO:3:

AATATCATTG GTGTTTCCTA T        21

-    INFORMATION FOR SEQ ID NO:4:

         (i) SEQUENCE CHARACTERISTICS:
             (A) LENGTH: 21 base pairs
             (B) TYPE: nucleic acid
             (C) STRANDEDNESS: single
             (D) TOPOLOGY: linear

        (ii) MOLECULE TYPE: DNA (genomic)


        (xi) SEQUENCE DESCRIPTION: SEQ ID NO:4:

TATCATCTTT GGTGTTTCCT A                                        21

-    INFORMATION FOR SEQ ID NO:5:

         (i) SEQUENCE CHARACTERISTICS:
             (A) LENGTH: 16 base pairs
             (B) TYPE: nucleic acid
             (C) STRANDEDNESS: single
             (D) TOPOLOGY: linear

        (ii) MOLECULE TYPE: DNA (genomic)


        (xi) SEQUENCE DESCRIPTION: SEQ ID NO:5:

GAGCAAGTGG TGTTGG                                              16

-    INFORMATION FOR SEQ ID NO:6:

         (i) SEQUENCE CHARACTERISTICS:
             (A) LENGTH: 40 base pairs
             (B) TYPE: nucleic acid
             (C) STRANDEDNESS: single
             (D) TOPOLOGY: linear

        (ii) MOLECULE TYPE: DNA (genomic)


        (xi) SEQUENCE DESCRIPTION: SEQ ID NO:6:

GTGCGCTTTT CCCAACACCA CCTGCTCCAA CCACCACCAG                    40

```
          —     INFORMATION FOR SEQ ID NO:7:

                (i)  SEQUENCE CHARACTERISTICS:
                     (A)  LENGTH: 30 base pairs
                     (B)  TYPE: nucleic acid
                     (C)  STRANDEDNESS: single
                     (D)  TOPOLOGY: linear

                (ii) MOLECULE TYPE: DNA (genomic)



                (xi) SEQUENCE DESCRIPTION: SEQ ID NO:7:

          CGCTTTTCCC AACACCATCT GCTCCAACCA                              30


          —     INFORMATION FOR SEQ ID NO:8:

                (i)  SEQUENCE CHARACTERISTICS:
                     (A)  LENGTH: 30 base pairs
                     (B)  TYPE: nucleic acid
                     (C)  STRANDEDNESS: single
                     (D)  TOPOLOGY: linear

                (ii) MOLECULE TYPE: DNA (genomic)



                (xi) SEQUENCE DESCRIPTION: SEQ ID NO:8:

          CGCTTTTCCC AACACCACTT GCTCCAACCA                              30
```

**Claims**

1. Oligonucleotide probe reagent consisting of a nylon membrane with a high density of anionic carboxyl groups to which membrane at least one oligonucleotide probe containing a 5' amine is covalently bound via an amide bond.

2. Probe reagent of claim 1, wherein the oligonucleotide probe contains a spacer.

3. Probe reagent of claim 1 or 2, wherein said nylon membrane is a Biodyne C™ membrane or an equivalent membrane thereto.

4. Probe reagent of claim 3, wherein a mixture of different probes is attached to said membrane at a discrete location.

5. Probe reagent of any one of claims 1 to 3, wherein a set of different probes is attached to said membrane so that each probe is present at a location on said membrane different from the location of every other member of said set.

6. Probe reagent of claim 5, wherein at least one probe of said set is a mixture of different probes.

7. Probe reagent of claim 5 or 6, wherein said set of probes is composed of probes that can hybridize to DNA that contains a CF mutation.

8. Probe reagent of claim 5 or 6, wherein said set of probes is composed of probes that can hybridize to DNA that contains a RAS mutation.

9. Probe reagent of claim 5 or 6, wherein said set of probes comprises probes for analysis of cytochrome P450 systems and chromosomal translations.

10. A method for constructing an oligonucleotide probe reagent according to any one of claims 1 to 9, said method comprising:

(a) activating a nylon membrane with a high density of anionic carboxyl groups by treatment with a reagent that converts carboxyl groups to O-acylureas; and

(b) reacting an oligonucleotide probe containing a 5' amine with an O-acylurea formed in step (a) to attach the probe to the membrane via an amide bond formed between said 5'-amine and said O-acylurea.

11. Method of claim 10, wherein said reagent that converts carboxyl groups to O-acylureas is EDC.

12. Method of claim 10 or 11, wherein after step (b), said membrane is treated to render inactive any unreacted O-acylureas formed in step (a) to a nucleophile.

13. Method of claim 12, wherein the treatment is performed with hydroxylamine and/or NaOH, preferably with 0.1N NaOH.

14. Kit, comprising reagents useful for performing the method according to any one of claims 10 to 13.

15. Kit, comprising amplification reagents for amplification of the nucleic acid(s) to be analyzed and the oligonucleotide probe reagent according to any one of claims 1 to 9 or reagents useful for performing the method according to any one of claims 10 to 13.

16. Kit of claim 15 comprising amplification reagents for PCR amplification.

FIG. 1

FIG. 2

Oligo Dilution Series
(Chemical Attachment)

Probe:  PA-1 PCR Product

[Oligo] in pm    5   4   3   2   1   0.5   0.25   0.12   0.06   0.03   0.01   0

N-ras 12 Asp

0S
1S
2S
3S

# FIG. 3

EP 0 511 559 A1

**a.** pmΔ508    0   0.1   0.2   0.5   1   0.1   0.2   0.5   1

●   ●   ●   ●   ●   ●   ●   ●

+ 10pm 508 Normal probe

**b.**

| | 1 | 2 | 3 | 4 | 5 | 6 | 7 | 8 |
|---|---|---|---|---|---|---|---|---|
| Δ508 | ● | ● | ● | ● | | ● | | |
| Normal 508 | | ● | ● | ● | ● | | | |
| Normal 551 | | | ● | ● | | | ● | |

*Fig. 4*

FIG. 5

## European Patent Office

**EUROPEAN SEARCH REPORT**

Application Number

## DOCUMENTS CONSIDERED TO BE RELEVANT

EP 92106603.1

| Category | Citation of document with indication, where appropriate, of relevant passages | Relevant to claim | CLASSIFICATION OF THE APPLICATION (Int. Cl.5) |
|---|---|---|---|
| A | EP - A - 0 416 801 (IMPERIAL CHEMICAL INDUSTRIES) * Claims * -- | 1-16 | C 12 Q 1/68 C 12 P 19/34 |
| P,A | WO - A - 91/12 343 (CETUS CORPORATION) * Abstract; claims * -- | 1-16 | |
| D,A | WO - A - 89/11 548 (CETUS CORPORATION) * Abstract; claims * ---- | 1-16 | |
| | | | **TECHNICAL FIELDS SEARCHED (Int. Cl.5)** |
| | | | C 12 Q C 12 P |

The present search report has been drawn up for all claims

| Place of search | Date of completion of the search | Examiner |
|---|---|---|
| VIENNA | 11-08-1992 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)